# EUROPÄISCHE PATENTANMELDUNG

(11) **EP 3 735 948 A1**
(43) Veröffentlichungstag der Anmeldung: **11.11.2020**
(21) Anmeldenummer: 20171819.4
(22) Anmeldetag: 28.04.2020
(51) Int. Cl.: A61F 13/00, A61L 15/46

(54) **WUNDAUFLAGE**

(30) Priorität: 10.05.2019 DE 102019112305
(71) Anmelder: Folwarzny, Alexander, 63457 Hanau (DE)
(72) Erfinder: FOLWARZNY, Alexander, 63457 Hanau (DE)
(74) Vertreter: Stoffregen, Hans-Herbert

(57) **Zusammenfassung**

Die Erfindung bezieht sich auf eine Wundauflage (10), umfassend eine ein- oder mehrlagige Aktivkohleschicht (12) mit einer wundseitig verlaufenden Vorderseite und einer wundabgewandten Rückseite, wobei auf der Vorderseite der Aktivkohleschicht (12) eine die Vorderseite ausschließlich bereichsweise abdeckende Abstandsstruktur (14) aus nicht-resorbierbarem Material aufgebracht ist.

## Beschreibung

Die Erfindung bezieht sich auf eine Wundauflage, umfassend eine ein- oder mehrlagige Aktivkohleschicht, wie Aktivkohlegewebe, -gewirk, -gelege oder -maschenware mit einer wundseitig verlaufenden Vorderseite und einer wundabgewandten Rückseite.

Der EP 0 749 295 B1 ist ein absorbierender Artikel, wie Damenbinde, zu entnehmen, der aus einer Öffnungen aufweisenden Oberschicht, einer flüssigkeitsundurchlässigen Unterschicht und einem absorbierenden Kern besteht. Der absorbierende Kern kann Aktivkohle enthalten.

Ein Wundverband nach der EP 0 026 572 B1 besteht aus einer Öffnung aufweisenden Heil- und Saugschicht, einer aus Kohlenstoffgewebe bestehenden angrenzenden Schicht sowie einer Rückseitenschicht.

Nach der WO 2013/028966 A2 ist eine Aktivkohleschicht zwischen zwei perforierten Schichten angeordnet, um eine Wundauflage zu bilden.

In der DE 10 2007 054 127 A1 wird ein Hygiene- oder Pflegeartikel beschrieben, der einen Wundexsudate absorbierenden Körper und eine Hülle umfasst, die aus durchlässigem Material besteht und z.B. ein Stanzmuster aufweisen kann.

Gegenstand der DE 10 2006 017 194 A1 ist ein Primärverband, der eine Beabstandung zu einem Sekundärverband ermöglicht. Der Sekundärverband weist Perforationen auf.

Nach dem Stand der Technik sind Wundauflagen vollflächig oder im erheblichen Umfang von einer wundseitig verlaufenden Schicht abgedeckt, so dass die Geruchs- und Bakterienbindung sowie Bindung anderer Pathogenen und Toxinen durch die Aktivkohleschicht beeinträchtigt wird.

Der vorliegenden Erfindung liegt die Aufgabe zugrunde, eine Wundauflage zuvor beschriebener Art so weiterzubilden, dass eine optimale Wirkung der Aktivkohlelage möglich ist, gleichzeitig jedoch sichergestellt ist, dass die Aktivkohleschicht an der Wunde nicht anhaften kann.

Zur Lösung der Aufgabe sieht die Erfindung vor, dass auf der Vorderseite der Aktivkohleschicht eine die Vorderseite ausschließlich bereichsweise abdeckende Abstandsstruktur aus nicht-resorbierbarem Material aufgebracht ist, die durch streifen- oder fadenförmige Gitter oder Netze und/oder wellenförmig oder zick-zack-förmig verlaufende Streifen oder Fäden und/oder punktförmige Erhebungen aus dem nicht-resorbierenden Material gebildet ist.

Erfindungsgemäß ist vorgesehen, dass die Aktivkohlelage nur in einem Umfang abgedeckt ist, dass ein unmittelbarer Kontakt mit einer Wunde unterbleibt. Somit können im erheblich größeren Umfang Geruch und Bakterien gebunden werden, als dies bei Wundauflagen nach dem Stand der Technik der Fall ist.

Die Fäden bzw. Streifen können einen Durchmesser D mit 0,5 mm ≤ D ≤ 1,0 mm, insbesondere 0,6 mm ≤ D ≤ 0,8 mm, aufweisen, ohne dass hierdurch die Erfindung eingeschränkt wird. Der Abstand der Fäden bzw. Streifen ist so zu wählen, dass die gewünschte unbedeckte effektive Fläche der Aktivkohleschicht zur Verfügung steht, insbesondere im Bereich zwischen 85% und 95 %.

Insbesondere beträgt der Abstand d der Fäden bzw. Streifen bei einem quadratischen Gitter 5 mm bis 10 mm, vorzugsweise 8 mm.

Die Abstandsstruktur kann bei Einsatz von fließfähigem Material durch Siebdruck oder auch durch 3D-Drucken auf die Aktivkohleschicht aufgebracht werden.

Um sicherzustellen, dass Reste von Kohlenstofffasern in die Wunde nicht eindringen können, die insbesondere von den Enden der Kohlenstofffasern stammen können, kann vorgesehen sein, dass die Wundauflage ein ausschließlich Rand der Aktivkohleschicht umlaufend abdeckendes Adhäsionsmaterial umfasst, wobei das Adhäsionsmaterial einen Rahmen bildet.

Die von dem Rahmen unbedeckte Vorderseite der Aktivkohleschicht ist als effektive Fläche der Wundauflage zu bezeichnen. Dabei sollte durch die Abstandsstruktur die effektive Fläche der Wundauflage maximal 50 %, vorzugsweise maximal 20 %, besonders bevorzugt maximal 15 % bis 5 %, abgedeckt werden.

Erfindungsgemäß wird eine Wundauflage zur Verfügung gestellt, die nahezu vollflächig wirkt, ohne dass die Gefahr besteht, dass ein Anhaften an einer Wunde erfolgt. Hierzu ist die Abstandsstruktur vorgesehen, die insbesondere durch ein Gitter oder ein Netz gebildet wird, das durch Streifen oder Fäden aus dem nicht-resorbierenden Material besteht. Andere Strukturen wie z.B. aus Streifen oder Fäden gebildete Wellen, Zick-Zack-Linien oder auch punktförmige Erhebungen aus dem nicht-resorbierenden Material sind gleichfalls möglich, um die Abstandsstruktur zu bilden.

Bei dem nicht-resorbierbaren Material handelt es sich insbesondere um Silikon oder um ein außenseitig silikonisiertes Material.

Es besteht auch die Möglichkeit, dass das nicht-resorbierende Material zumindest ein Material aus der Gruppe Polyamid, Polyester, Polypropylen, Polyvinylidenfluorid, Polytetrafluorethylen, Seide, Metalldraht, wie titanbeschichteter Stahldraht, ist.

Das den Rand der Aktivkohleschicht abdeckende Material besteht insbesondere auch aus Silikon oder einem silikonisierten Material. Andere geeignete im Medizinbereich zum Einsatz gelangende Adhäsionsmaterialien wie z.B. solche auf der Basis von Polyacrylat kommen gleichfalls in Frage.

Insbesondere ist vorgesehen, dass sich der Rahmen entlang der Rückseite der Kohlenstoffschicht erstreckt, so dass die effektive Fläche gleich der der Fläche der Aktivkohleschicht ist.

Es besteht jedoch auch die Möglichkeit, das Adhäsionsmaterial auf der Vorderseite aufzubringen.

Auch kann in Weiterbildung der Erfindung das Adhäsionsmaterial ein Bereich der Abstandsstruktur sein. Hierzu werden die freien Enden der Aktivkohlefäden von einem einen Rahmen bildenden Streifen aus dem nicht-resorbierenden Material abgedeckt, wobei die Streifen gleichzeitig die Klebewirkung auf einer Haut sicherstellen.

Selbstverständlich besteht auch die Möglichkeit, dass sich das den Rahmen bildende Adhäsionsmaterial sowohl entlang Vorder- als auch Rückseite der Aktivkohleschicht erstreckt.

Ferner besteht die Möglichkeit, dass die Rückseite der Aktivkohleschicht von einem absorbierenden Saugkörper abgedeckt ist.

Voraussetzung für das nicht-resorbierbare Material ist, dass dieses mit der Aktivkohleschicht im erforderlichen Umfang verbunden ist, so dass ein Lösen bei der Nutzung ausgeschlossen ist.

Kann der Rand der Wundauflage von einem Adhäsionsmaterial abgedeckt sein, so ist dies kein zwingendes Merkmal. Vielmehr ist insbesondere vorgesehen, dass sich entlang wundseitiger Außenfläche der Aktivkohleschicht ausschließlich die Abstandsstruktur erstreckt.

Mit anderen Worten erstreckt sich entlang wundseitig verlaufender Außenfläche der Aktivkohleschicht ausschließlich die Abstandstruktur. Die Wundauflage kann sodann in geeigneterweise auf eine Körperfläche z.B. mit einem gesonderten Verband fixiert werden.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von bevorzugten Ausführungsbeispielen.

Es zeigen:
- Fig. 1: eine Explosionsdarstellung einer ersten Ausführungsform einer Wundauflage,
- Fig. 2.: die Wundauflage gemäß Fig. 1 in Draufsicht,
- Fig. 3: die Wundauflage gemäß Fig. 1 und 2 in auseinandergezogener Darstellung,
- Fig. 4.: eine Explosionsdarstellung einer zweiten Ausführungsform einer Wundauflage,
- Fig. 5: die Wundauflage gemäß Fig. 4 in Draufsicht,
- Fig. 6: die Wundauflage gemäß der Fig. 4 und 5 in Rückansicht,
- Fig. 7: die Wundauflage gemäß Fig. 4 bis 6 in auseinandergezogener Darstellung,
- Fig. 8: Prinzipdarstellungen von Draufsichten von Wundauflagen mit Abstandsstrukturen aus nicht-resorbierbarem Material,
- Fig. 9: eine Explosionsdarstellung einer dritten Ausführungsform einer Wundauflage,
- Fig. 10: die Wundauflage gemäß Fig. 9 in Draufsicht,
- Fig. 11: die Wundauflage gemäß Fig. 9 und 10 in Rückansicht,
- Fig. 12: die Wundauflage gemäß Fig. 9 bis 11 in auseinandergezogener Darstellung,
- Fig. 13: eine vierte Ausführungsform einer Wundauflage in Explosionsdarstellung,
- Fig. 14: die Wundauflage gemäß Fig. 13 in Draufsicht,
- Fig. 15: die Wundauflage gemäß Fig. 13 und 14 in Rückansicht,
- Fig. 16: die Wundauflage gemäß Fig. 13 bis 15 in auseinandergezogener Darstellung,
- Fig. 17: eine fünfte Ausführungsform einer Wundauflage in Explosionsdarstellung,
- Fig. 18: die Wundauflage gemäß Fig. 17 in Draufsicht und
- Fig. 19: die Wundauflage gemäß Fig. 17 und 18 in auseinandergezogener Darstellung.

Den Figuren sind verschiedene Ausführungsformen von Wundauflagen zu entnehmen, wobei grundsätzlich für gleiche Elemente gleiche Bezugszeichen verwendet werden.

Kennzeichnend für sämtliche Wundauflagen ist, dass eine zur Geruchs- und Bakterienbindung sowie Bindung anderer Pathogenen und Toxinen verwendete Aktivkohleschicht nur im geringen Umfang abgedeckt ist, d.h., dass vorzugsweise bis zu 90 bis 95 % der Fläche wirken kann. Um jedoch ein Anhaften der Aktivkohleschicht an einer Wunde zu vermeiden, ist eine Abstandsstruktur vorgesehen, die wundseitig auf der Aktivkohleschicht aufgebracht ist.

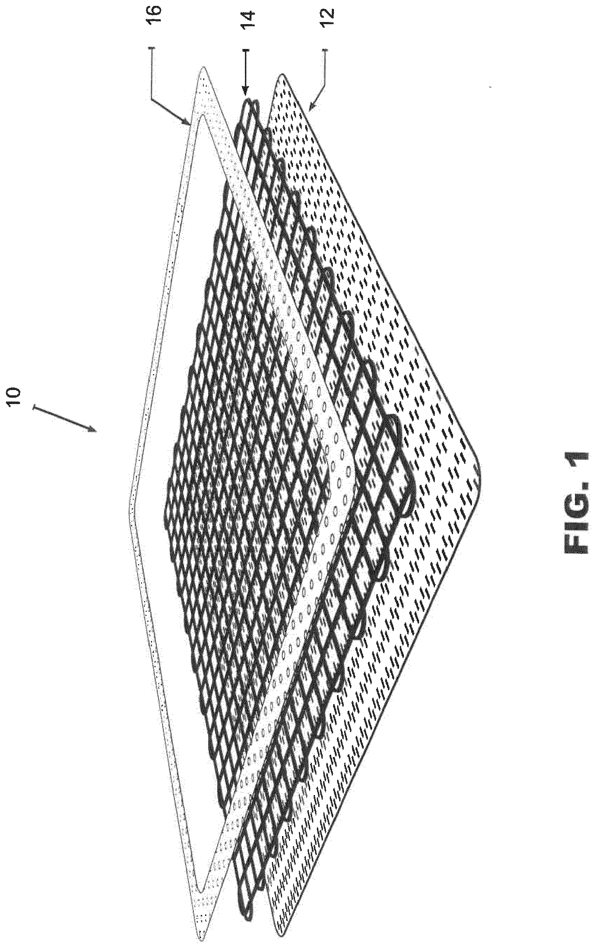

Den Fig. 1 bis 3 ist eine erste Ausführungsform einer Wundauflage 10 zu entnehmen, die eine Aktivkohleschicht 12 aufweist, die ein- oder mehrlagig ausgebildet sein kann.

Die Aktivkohleschicht 12 besteht aus Kohlenstofffasern, aus denen z.B. ein Gewebe, ein Gewirk, ein Gelege oder eine Maschenware zur Bildung der Aktivkohleschicht 12 ausgebildet ist.

Um einen Kontakt zwischen der Aktivkohleschicht 12 und einer Wunde auszuschließen, ist auf der einer Wunde zugewandten Seite der Aktivkohleschicht 12, also der Vorderseite, eine die Aktivkohleschicht 12 ausschließlich bereichsweise abdeckende Abstandsstruktur aufgebracht, die im Ausführungsbeispiel die Form eines Gitters 14 besitzt. Dieses kann z.B. durch Siebdruck oder 3D-Drucken auf die Aktivkohleschicht 12 aufgebracht sein. Andere geeignete Verfahren sind gleichfalls möglich.

Das Gitter 14 besteht aus Fäden oder Streifen, die einen Durchmesser von vorzugsweite 0,5 mm und 1,0 mm, insbesondere im Bereich zwischen 0,6 mm und 0,8 mm, aufweisen. Der Abstand zwischen den parallel zueinander verlaufenden Längs- und Querfäden bzw. Streifen beträgt zwischen 5 mm und 10 mm.

Insbesondere sind die Durchmesser der Fäden- bzw. Streifen zum Abstand zwischen diesen derart zueinander abgestimmt, dass die wirksame wundseitig verlaufende von einer einen Rahmen 16 bildenden Klebeschicht nicht abgedeckte effektive Aktivkohlenschichtfläche weniger als 50 %, bevorzugterweise weniger als 20 %, insbesondere weniger als 15 - 5 %, von dem Gitter 14 abgedeckt ist.

Das Gitter 14 besteht aus einem nicht-resorbierbaren Material, insbesondere aus Silikon oder einem Material, das umfangsseitig silikonisiert ist.

Materialien wie Polyamid, Polyester, Polypropylen, Polyvinylidenfluorid, Polytetrafluorethylen, Seide, Metalldraht, wie titanbeschichteter Stahldraht, kämen gleichfalls in Frage. Voraussetzung ist jedoch, dass diese auf der Aktivkohleschicht 12 fixiert sind.

Um die Wundauflage 10 klebend auf eine Haut zu fixieren, ist eine aus Klebstoffmaterial bestehende wundseitig verlaufende den Rahmen 16 bildende Schicht z.B. aus Silikon, Polyacrylat oder anderen geeignetem Material aufgebracht, das die erforderliche Klebewirkung sicherstellt. Durch den Rahmen 16 wird gleichzeitig der Rand der Aktivkohleschicht 12 abgedeckt, so dass Faser- bzw. Kohlenstoffreste gebunden werden und somit nicht in eine Wunde eindringen können.

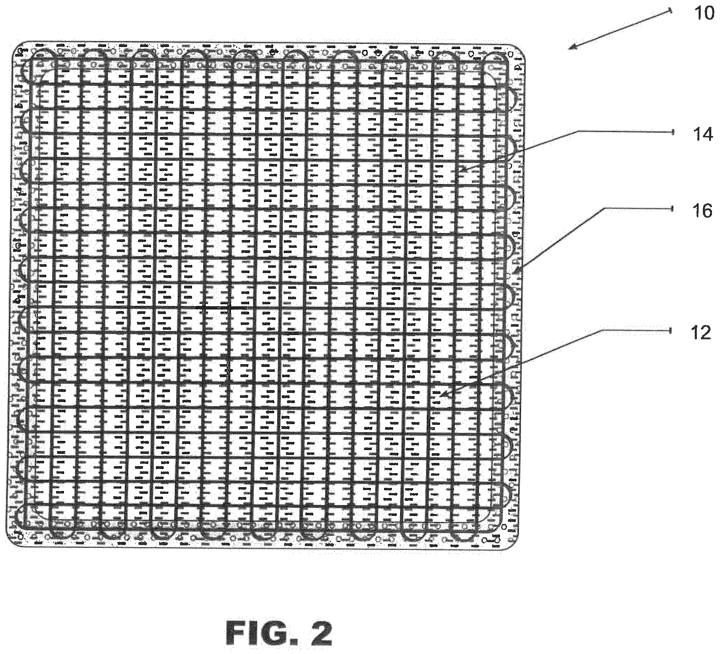

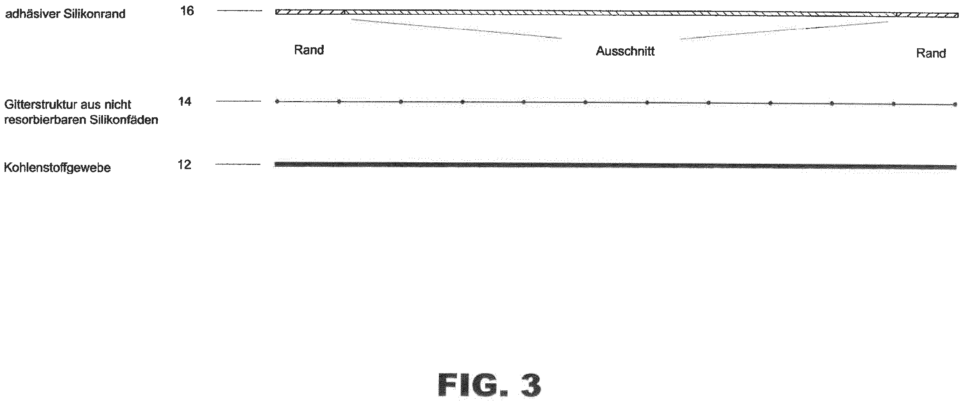

Die Wundauflage 10 ist in Fig. 2 in Draufsicht dargestellt. Dabei wird erkennbar, dass der Rahmen 16 quasi außenseitig bündig mit dem Rand der Aktivkohleschicht 12 abschließt, also nicht über den Rand der Aktivkohleschicht 12 seitlich vorsteht. Dies wird insbesondere auch durch die Fig. 3 verdeutlicht.

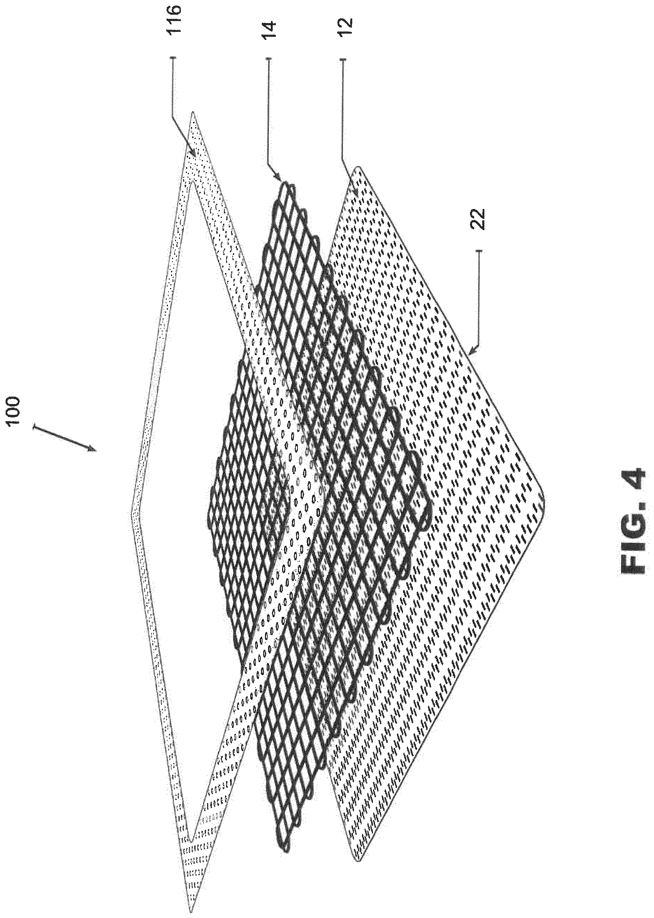

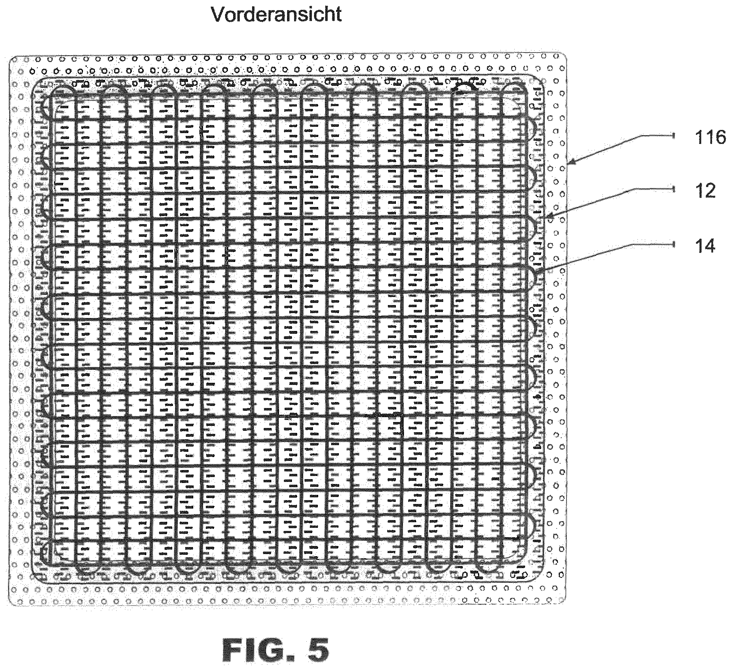

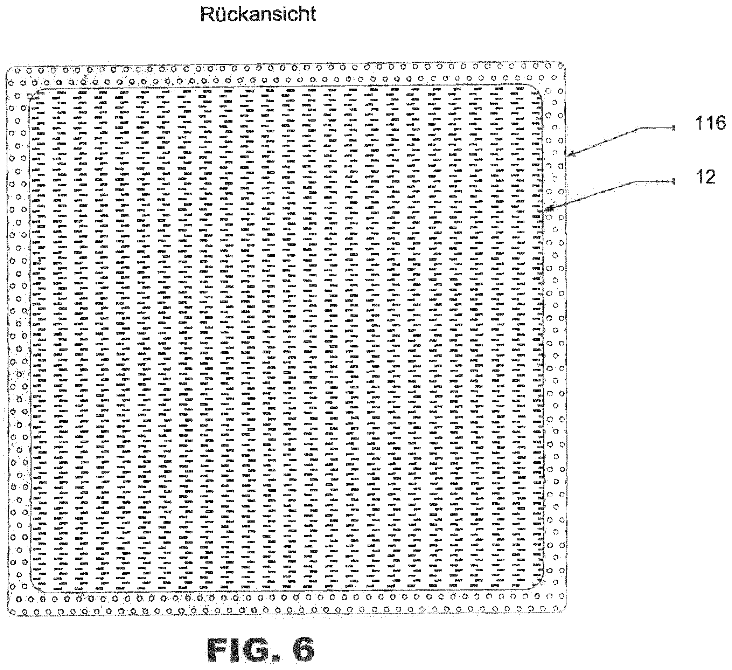

Eine zweite Ausführungsform einer Wundauflage 100 ist den Fig. 4 bis 7 zu entnehmen. Dabei unterscheidet sich diese von der der Fig. 1 bis 3 dahingehend, dass der insbesondere aus Silikonmaterial bestehende Rahmen 116 sich über den Umfangsrand 22 der Aktivkohleschicht 12 erstreckt, wie sich aus den Fig. 5, 6 und 7 ergibt.

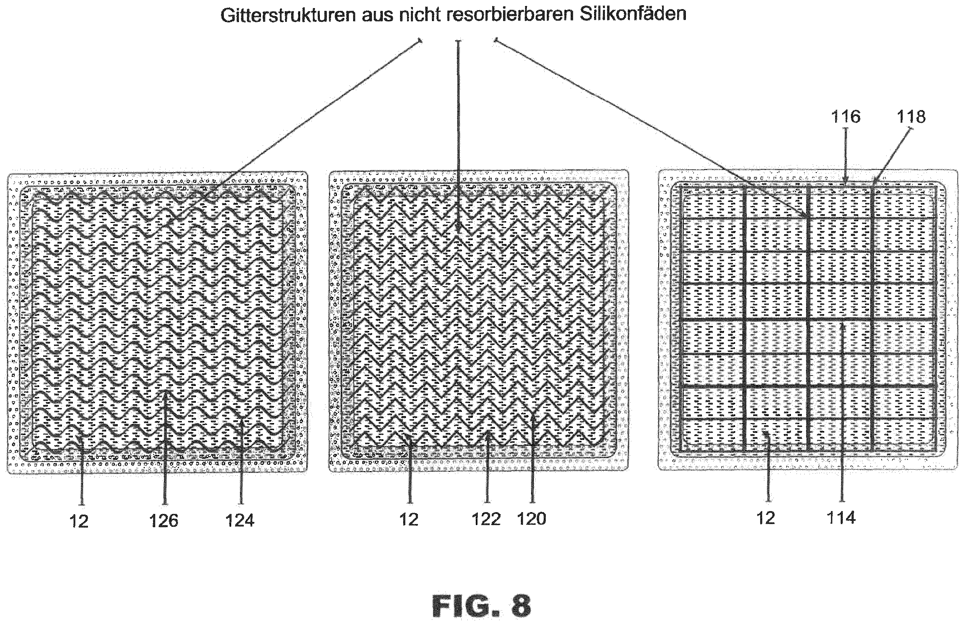

Unabhängig hiervon weist auch im Ausführungsbeispiel der Fig. 4 bis 7 die Abstandsstruktur die Form eines Gitters 14 auf, ohne dass hierdurch die erfindungsgemäße Lehre eingeschränkt wird. Vielmehr können auch andere Abstandsstrukturen gewählt werden, wie diese beispielhaft der Fig. 8 zu entnehmen ist. So kann eine Gitterstruktur 114 vorgesehen sein, die sich durch vertikalschneidende Streifen oder Fäden 116, 118 auszeichnet, die ein Rechteck einschließen (rechte Darstellung von Fig. 8). Dabei können die Streifen bzw. Fäden voneinander abweichende Durchmesser aufweisen. Auch können einige der Fäden bzw. Streifen eine Stärke aufweisen, dass eine Klebewirkung erzielt wird.

In der mittleren Darstellung der Fig. 8 wird eine Abstandsstruktur durch zickzackförmig verlaufende Fäden bzw. Streifen 120, 122 erzielt, die parallel zueinander verlaufen.

In der linken Darstellung der Fig. 8 sind Streifen bzw. Fäden 124, 126 wellenförmiger Geometrie zur Bildung der gewünschten Abstandsstruktur auf der Aktivkohleschicht 12 aufgebracht.

Auch durch ein Punktemuster kann eine Abstandsstruktur gebildet werden.

Unabhängig hiervon ist erkennbar, dass die Aktivkohleschicht 12 randseitig von einem eine Klebewirkung erzielenden Rahmen abgedeckt ist, wie dies zuvor erläutert worden ist.

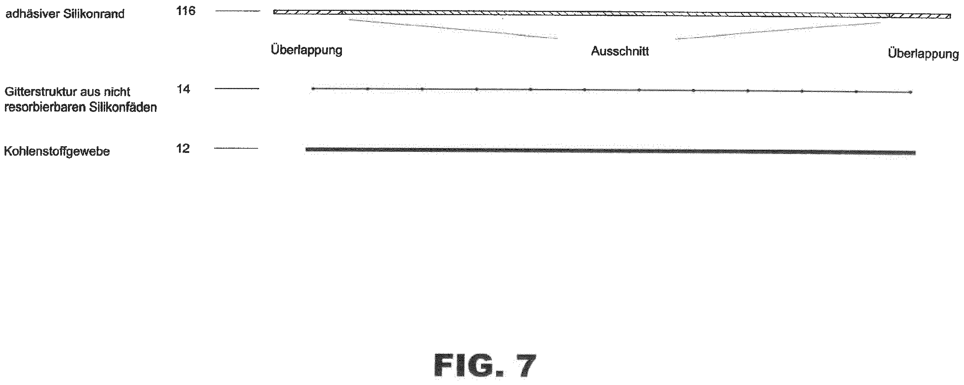

Wie sich aus der Fig. 7 ergibt, ist der Rahmen 116 geometrisch derart an die Aktivkohleschicht 12 angepasst, dass ersterer teilweise innerhalb der Aktivkohleschicht 12 und teilweise außerhalb von dieser verläuft, wie insbesondere aus der Fig. 7 erkennbar ist.

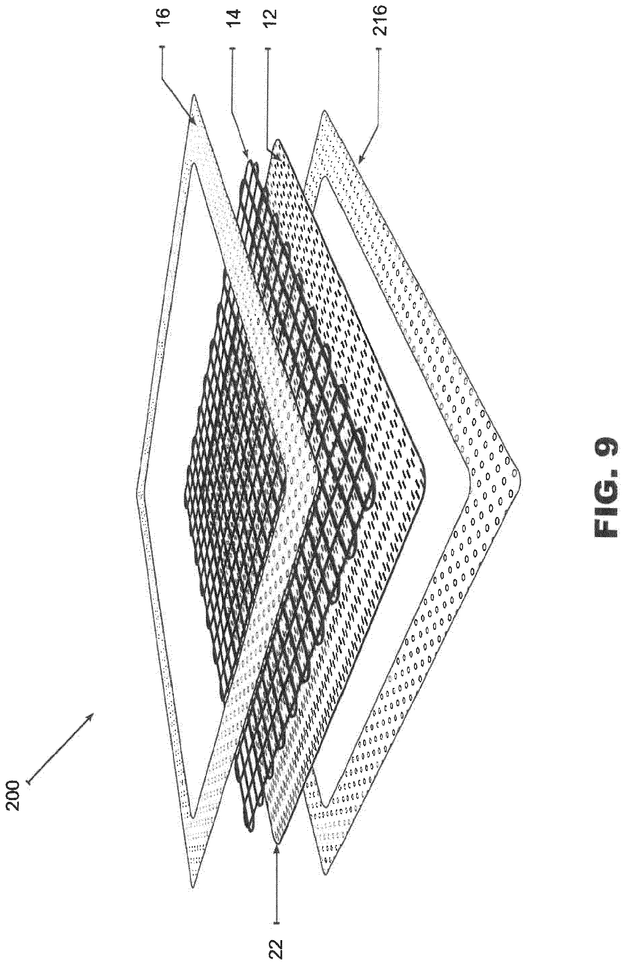

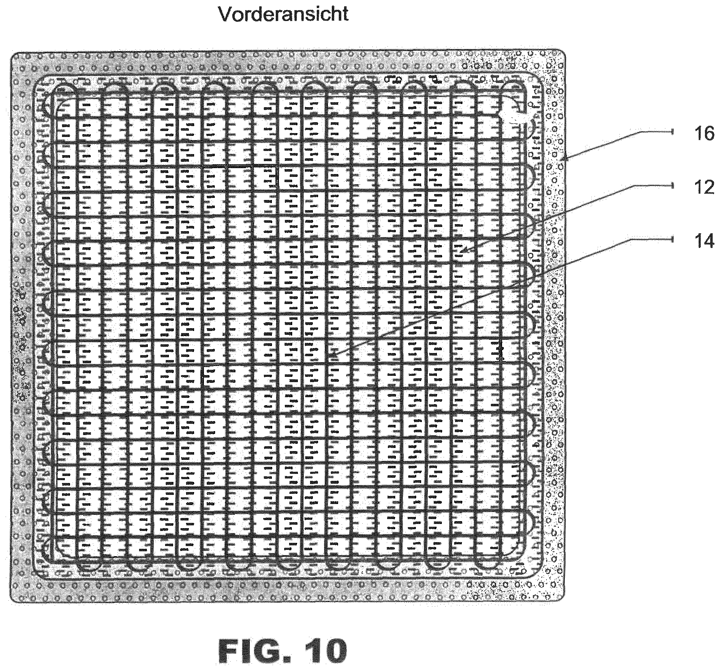

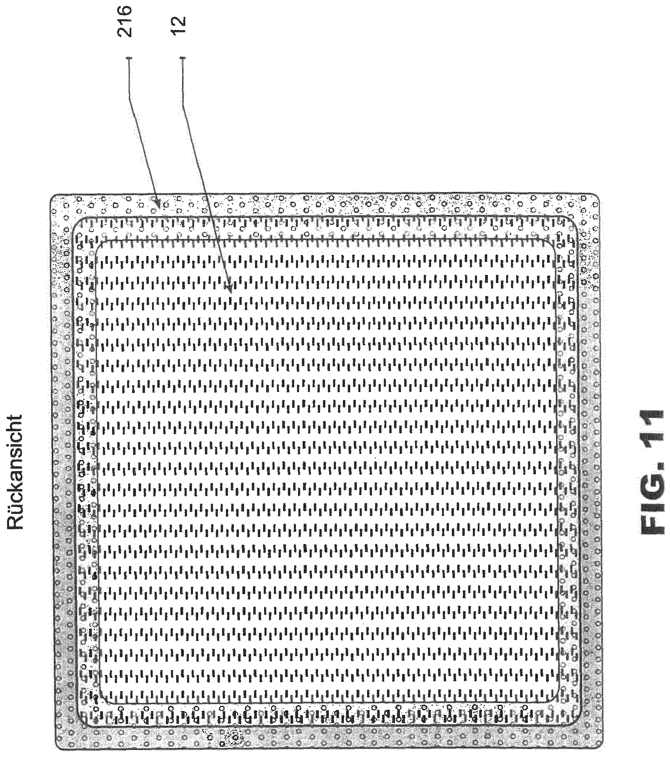

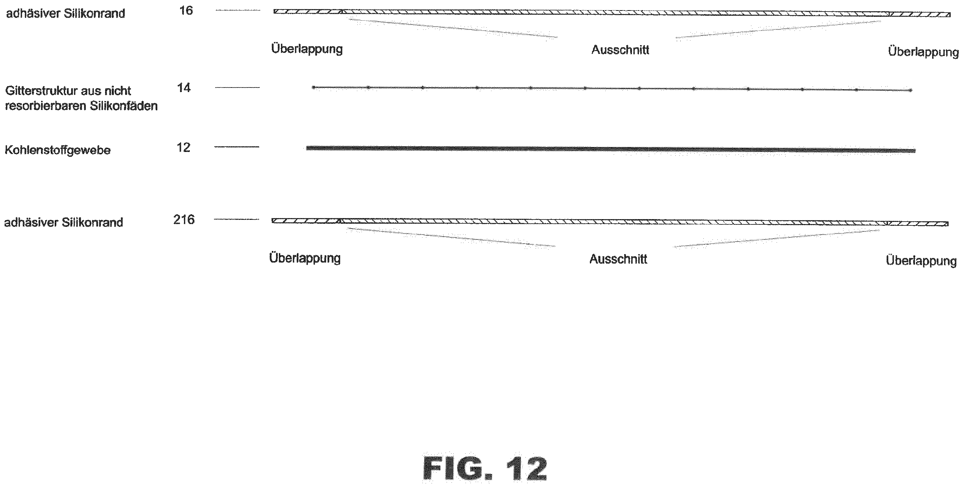

Eine weitere Ausführungsform einer Wundauflage 200 ist den Fig. 9 bis 12 zu entnehmen. Diese unterscheidet sich von den vorhergehenden dahingehend, dass sowohl entlang der Vorder- als auch der Rückseite der Aktivkohleschicht 12 ein Rahmenelement 16, 216 vorgesehen ist, das den umlaufenden Rand 22 der Aktivkohleschicht 12 abdeckt. Dabei wird - wie bei den vorhergehenden Ausführungsbeispielen - auch der Randbereich der Abstandsstruktur abgedeckt, der bei der Wundauflage 200 gleichfalls als Gitter ausgebildet ist. Der Aufbau ergibt sich insbesondere auch aus der Fig. 12.

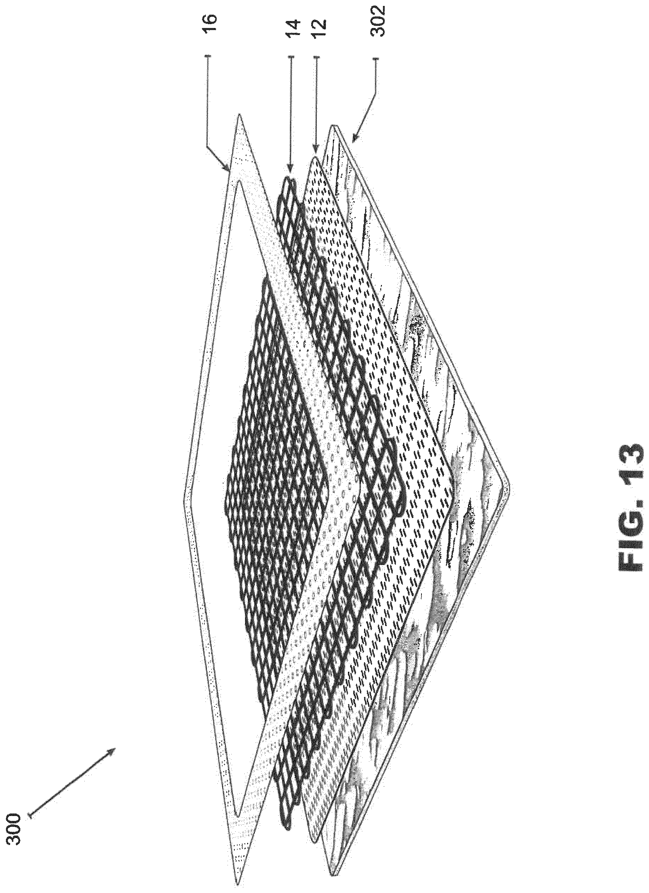

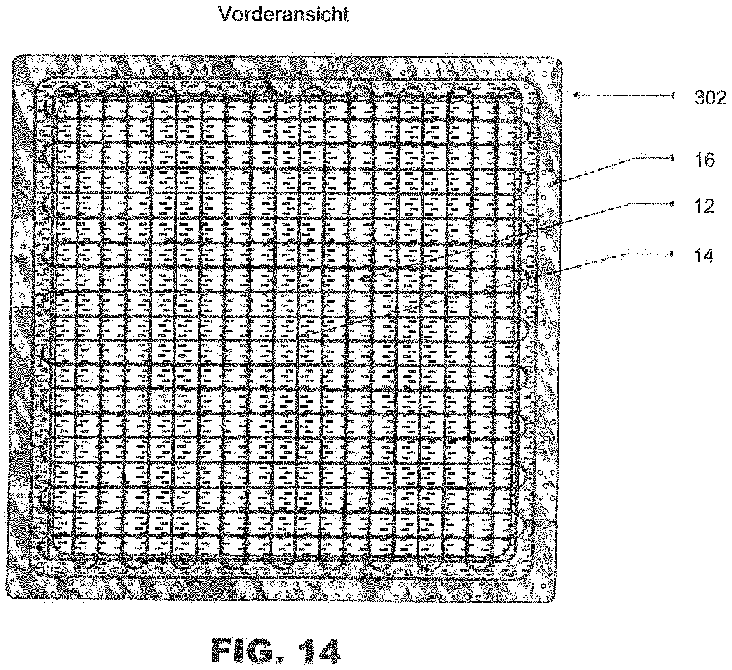

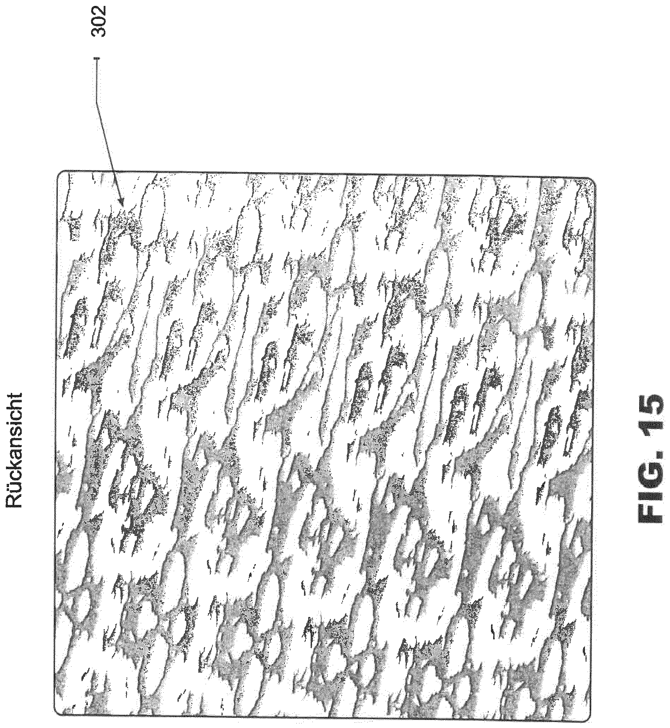

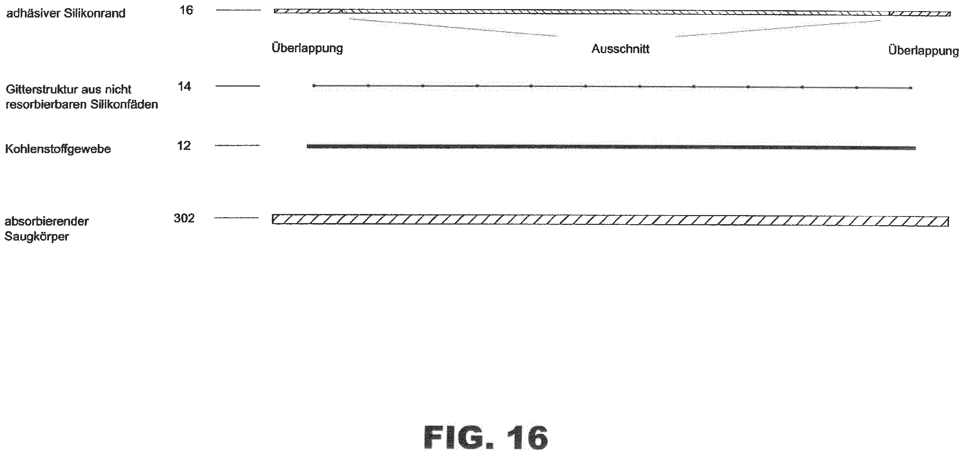

Eine Wundauflage 300 gemäß der Fig. 13 bis 16 zeichnet sich dadurch aus, dass diese rückseitig von einer absorbierbaren flächigen Schicht 302 abgedeckt ist, die mittels der als Rahmen ausgebildeten Klebeschicht 16 fixiert ist. Ansonsten ergibt sich ein Aufbau, wie dieser im Zusammenhang mit den Ausführungsformen der Fig. 1 bis 12 erläutert worden ist.

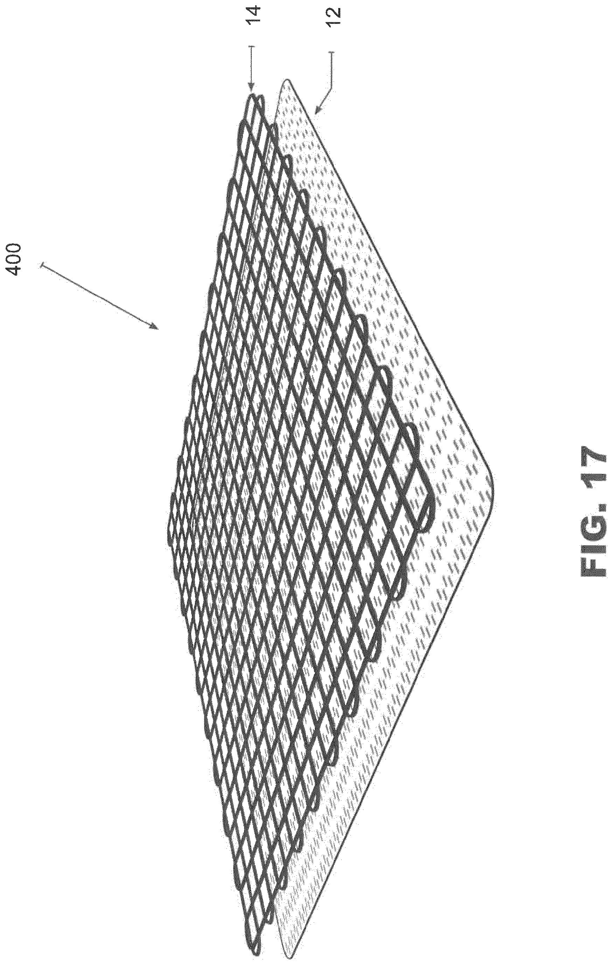

Den Fig. 17 bis 19 ist eine weitere hervorzuhebende Ausführungsform einer erfindungsgemäßen Wundauflage 400 zu entnehmen, die ebenfalls selbsterklärend ist.

So ist in Fig. 17 die Aktivkohleschicht 12 und die sich entlang dieser erstreckende, jedoch zeichnerisch beabstandete Abstandsstruktur in Form des Gitters 14 dargestellt, das aus dem nicht-resorbierenden Material, insbesondere aus Silikon oder einem Material, das umfangsseitig silikonisiert ist, besteht.

Ist in den Fig., die die Aktivkohlesicht 12 abdeckende Abstandsstruktur in Gitterform dargestellt, so sind andere Geometrien wie Netze oder wellenförmige oder zick-zackförmige Streifen oder Fäden gleichfalls möglich, die die Aktivkohlesicht 12, bevorzugt max. 15 % bis 5 %, abdecken sollte. Hierdurch erfolgt jedoch keine Einschränkung der erfindungsgemäßen Lehre.

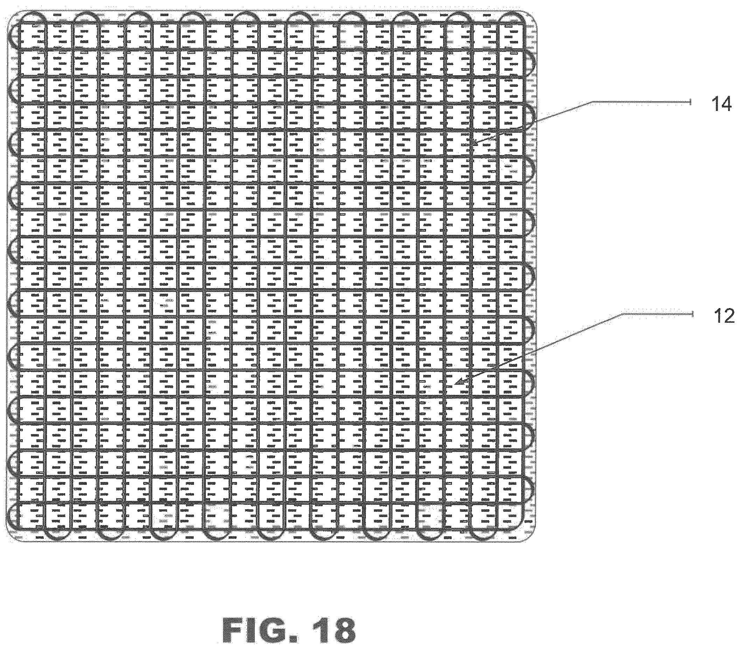

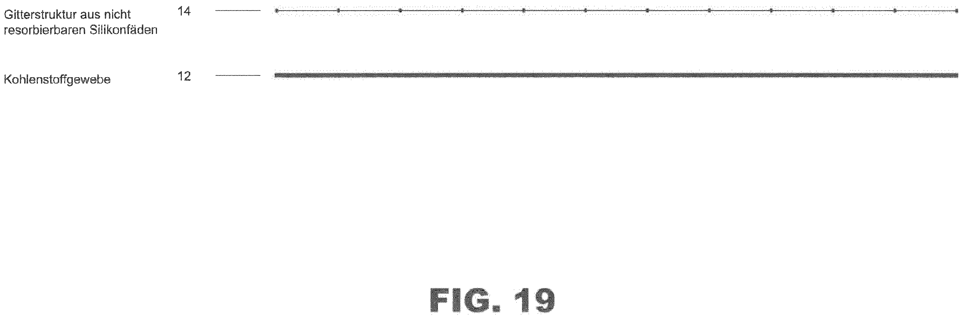

In der Fig. 18 ist eine Draufsicht der Wundauflage 400 dargestellt. Man erkennt, dass sich das Gitter 14 bis zum Rand der Aktivkohleschicht 12 erstreckt, so dass eine gesonderte Randabdeckung vorliegt. Gleiches ergibt sich aus der Darstellung gemäß Fig. 19.

## Patentansprüche

1. Wundauflage (10, 100, 200, 300, 400), umfassend eine ein- oder mehrlagige Aktivkohleschicht (12), wie Aktivkohlegewebe, -gewirk, -gelege oder
- maschenware, mit einer wundseitig verlaufenden Vorderseite und einer wundabgewandten Rückseite,
**dadurch gekennzeichnet,**
**dass** auf der Vorderseite der Aktivkohleschicht (12) eine die Vorderseite ausschließlich bereichsweise abdeckende Abstandsstruktur (14) aus nicht-resorbierbarem Material aufgebracht ist, die durch streifen- oder fadenförmige Gitter oder Netze und/oder wellenförmig oder zick-zack-förmig verlaufende Streifen (116, 118, 120, 122, 124, 126) oder Fäden und/oder punktförmige Erhebungen aus dem nicht-resorbierenden Material gebildet ist.

2. Wundauflage nach Anspruch 1,
**dadurch gekennzeichnet,**
**dass** die Fäden bzw. Streifen (114, 116, 118, 120, 122, 124, 126) einen Durchmesser D mit 0,5 mm ≤ D ≤ 1,0 mm, insbesondere 0,6 mm ≤ D ≤ 0,8 mm, aufweisen.

3. Wundauflage nach Anspruch 1 oder 2,
dass sich entlang wundseitiger Außenfläche der Aktivkohleschicht (12) ausschließlich die Abstandsstruktur (14) erstreckt.

4. Wundauflage nach zumindest einem der vorhergehenden Ansprüche,
**dadurch gekennzeichnet,**
**dass** die Abstandsstruktur (14) weniger als 20 %, vorzugsweise max. 15 %, besonders bevorzugt 15 % bis 5 %, der Aktivkohlesicht (12) abdeckt.

5. Wundauflage nach zumindest einem der vorhergehenden Ansprüche,
**dadurch gekennzeichnet,**
**dass** die Wundauflage (10, 100, 200, 300) einen Rand der Aktivkohleschicht (12) umlaufend abdeckendes Adhäsionsmaterial (16) umfasst.

6. Wundauflage nach zumindest einem der vorhergehenden Ansprüche,
**dadurch gekennzeichnet,**
**dass** das Adhäsionsmaterial einen Rahmen (16) bildet und dass von dem Rahmen unbedeckte Vorderseite der Aktivkohleschicht (12) effektive Fläche der Wundauflage ist, die flächenmäßig maximal 50 %, vorzugsweise maximal 20 %, besonders bevorzugt maximal 15 % bis 5 % von der Abstandsstruktur (14) abgedeckt ist.

7. Wundauflage nach zumindest einem der vorhergehenden Ansprüche,
**dadurch gekennzeichnet,**
**dass** das nicht-resorbierende Material Silikon oder außenseitig silikonisiertes Material ist.

8. Wundauflage nach zumindest einem der vorhergehenden Ansprüche,
**dadurch gekennzeichnet,**
**dass** das nicht-resorbierende Material zumindest ein Material aus der Gruppe Polyamid, Polyester, Polypropylen, Polyvinylidenfluorid, Polytetrafluorethylen, Seide, Metalldraht, wie titanbeschichteter Stahldraht, ist.

9. Wundauflage nach zumindest einem der vorhergehenden Ansprüche,
**dadurch gekennzeichnet,**
**dass** der Rahmen sich entlang Vorder- und/oder Rückseite der Aktivkohleschicht (12) erstreckt.

10. Wundauflage nach zumindest einem der vorhergehenden Ansprüche,
**dadurch gekennzeichnet,**
**dass** die Aktivkohleschicht (12) rückseitig von einer absorbierenden Schicht (302) abgedeckt ist.
